# EUROPEAN PATENT SPECIFICATION

(11) **EP 2 727 539 B1**
(45) Date of publication and mention of the grant of the patent: **17.01.2018**
(21) Application number: 13190087.0
(22) Date of filing: 24.10.2013
(51) Int. Cl.: A61B 17/04, A61F 2/00, A61B 17/00, A61B 17/06, A61B 90/00

(54) **Surgical system including an anchor insertable into a cannula of an introducer**
Chirurgisches System mit einem in eine Kanüle einführbaren Anker einer Einführvorrichtung
Système chirurgical comprenant un élément d'ancrage pouvant être inséré dans une canule d'un dispositif d'introduction

(30) Priority: 02.11.2012 US 201261721563 P; 30.09.2013 US 201361884142 P; 10.10.2013 US 201314050352
(43) Date of publication of application: 07.05.2014
(73) Proprietor: Coloplast A/S, 3050 Humlebaek (DK)
(72) Inventor: Deitch, Sarah J., Minneapolis, MN Minnesota 55417 (US); Witzmann, Michael M., Shoreview, MN Minnesota 55126 (US)

(56) References cited:
- EP-A2- 1 484 022
- US-A1- 2004 002 734
- US-A1- 2011 022 061

## Description

### Background

Intracorporeal suturing of tissue during surgery presents challenges to the surgeon in that the surgeon is called upon to manipulate suturing instruments within the confines of a relatively small incision formed in the patient's body. In some cases, the surgeon is unable to see the suture site. In such a case, the surgeon will digitally palpate with a finger to locate a landmark within the intracorporeal site, and then deliver the suture near at or near the landmark. Tying of the suture inside the patient at the intracorporeal site can be challenging since the surgeon is unable to see the site.

US 2004/0002734 A1 discloses a suture anchor delivery system including a handle having a needle extending therefrom. A suture anchor assembly is slidably received on the needle. The suture anchor assembly includes a proximal anchor, a distal anchor and a suture extending therebetween.

Improved suturing instruments and improved methods of delivering sutures would be welcomed by the surgical staff.

### Summary

One aspect provides a surgical system including an anchor and an introducer provided to deliver the anchor into tissue. The invention is further defined in claim 1.

### Brief Description of the Drawings

The accompanying drawings are included to provide a further understanding of embodiments and are incorporated in and constitute a part of this specification. The drawings illustrate embodiments and together with the description serve to explain principles of embodiments. Other embodiments and many of the intended advantages of embodiments will be readily appreciated as they become better understood by reference to the following detailed description. The elements of the drawings are not necessarily to scale relative to each other. Like reference numerals designate corresponding similar parts.
Figure 1 is a perspective view of one embodiment of a surgical system including an anchor that is insertable into a cannula of an introducer.
Figure 2A is a perspective view of the anchor illustrated in Figure 1.
Figure 2B is an end view of the anchor.
Figure 2C is a perspective view of another example of an anchor.
Figure 3 is a perspective view of the anchor outside of the cannula illustrated in Figure 1.
Figure 4 is a perspective view of the anchor inserted into a lumen of the cannula illustrated in Figure 1.
Figure 5 is a schematic view of one embodiment of the surgical system provided to anchor a support material to tissue of the human body, with the support material having an arm inserted through each of two obturator foramen of the pelvis.
Figure 6 is a schematic view of one embodiment of the surgical system employed to anchor a support material to the tissue of the human body showing a pre-pubic portion being attached to the periosteum of the pubic bone.
Figure 7 is a schematic view of the anchor illustrated in Figure 1 secured to tissue with a stopper coupled with a suture and located between the anchor and a slip knot.
Figure 8 is a schematic view of two anchors as illustrated in Figure 1 secured to tissue and coupled with a suture.

### Detailed Description

In the following Detailed Description, reference is made to the accompanying drawings, which form a part hereof, and in which is shown by way of illustration specific embodiments in which the invention may be practiced. In this regard, directional terminology, such as "top," "bottom," "front," "back," "leading," "trailing," etc., is used with reference to the orientation of the Figure(s) being described. Because components of embodiments can be positioned in a number of different orientations, the directional terminology is used for purposes of illustration and is in no way limiting. It is to be understood that other embodiments may be utilized and structural or logical changes may be made without departing from the scope of the present invention. The following detailed description, therefore, is not to be taken in a limiting sense, and the scope of the present invention is defined by the appended claims.

The features of the various exemplary embodiments described in this application are suitable and intended to be combined with each other, unless specifically noted otherwise.

Anterior means "forward" or "front," and posterior means "rearward" or "back." Relative to surfaces of an organ in the human body, an anterior surface of an instrument inserted into the organ will be oriented forward toward the belly and a posterior surface will be oriented rearward toward the spine.

End means an end most location and end portion means that segment adjacent to and near the end of an object. For example, two opposing ends of an object are each equidistant from a mid-point of the object and between the mid-point and each end of the object is an end portion of the object.

Embodiments provide a surgical system including an introducer that is configured to deliver an anchor to an intracorporeal tissue site. The introducer includes a cannula that allows placement of an anchor at a landmark in tissue deep within an incision site, which may be out of the field of vision of the surgeon. The anchor is configured to be secured within the cannula so that it does not rotate or fall out of the cannula during insertion into the tissue. A length of suture is provided that is attached to the anchor, where the suture may be tied or otherwise terminated to itself outside of the incision site and then subsequently directed to the intracorporeal landmark.

Some incontinence treatment devices have several arms, including some form of arms that traverse the obturator foramen (called transobturator arms) and other arms that are implanted anterior to the pubic bone (called pre-pubic arms). A first set of tools is used to place the transobturator arms and a second, different set of tools is used to place the pre-pubic arms. The pre-pubic arms are tunneled anterior to the pelvis and exit the skin of the abdomen.

In contrast, embodiments of the system described in this specification provide a support with two transobturator arms and a system to attach a portion of the support directly and efficiently to the periosteum tissue. The system obviates the use of additional pre-pubic arms and additional tools that tunnel the pre-pubic arms under the skin. The system is easier to implant compared to a four arm or six arm support, and reduces the amount of time that the patient is in the operating room.

One approach to treating urinary incontinence places a support inferior to the urethra and directs arms upward from the support alongside the bladder along a U-shaped pathway. A significant advance over the U-shaped pathway was provided by Dr. Emmanuel Delorme as described in his U.S. Pat. No. 6,638,211 and included placing arms of a support through the obturator foramen along a V-shaped pathway. This application provides another advance in supporting the pelvic anatomy by recognizing that support material can be robustly attached to the periosteum tissue through the use of an anchoring system. The anchoring system allows the surgeon to place the support inside of the patient and directly fixate the support to periosteum tissue that is present over the exterior of the bones. This approach does away with needles and other tools that tunnel the arms of a support through tissue. The anchoring system described in this application is compatible with a true single (only one) incision formed in the patient.

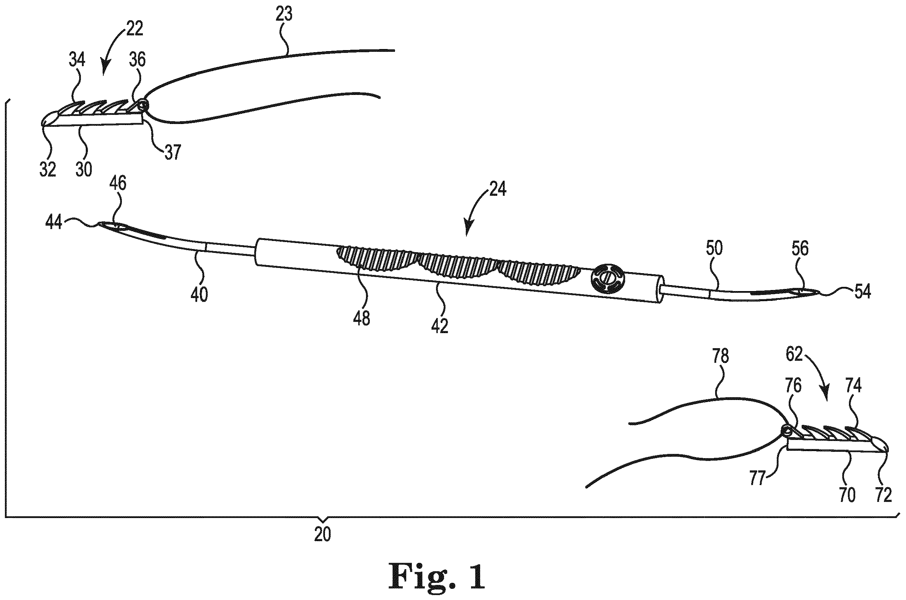

Figure 1 is a perspective view of one embodiment of a surgical system 20. The surgical system 20 (system 20) includes an anchor 22 attached to a length of suture 23 and an introducer 24 adapted to deliver the anchor 22 to an intracorporeal landmark. The anchor 22 is sized to be inserted into the introducer 24, and the introducer 24 is sized to be inserted through a single incision to push or direct the anchor 22 into tissue. The suture 23 trails behind the anchor 22 and is available for subsequent ligation of the tissue, or for subsequent attachment of a support to the tissue.

The anchor 22 includes a body 30 having a pointed leading end 32 that is configured to pierce tissue, a spine 34 projecting radially away from the body 30 and configured to engage with or anchor to tissue, and an eyelet 36 attached to a trailing end 37 of the body 30. The length of suture 23 is inserted through the eyelet 36.

The introducer 24 includes a cannula 40 extending from a handle 42. The cannula 40 has a pointed distal end 44 and an opening 46 formed in the cannula 40. The opening 46 or lumen 46 is sized to receive the body 30 of the anchor 22. The handle 42 includes a gripping surface 48 formed on at least one side of the handle 42. It is acceptable to provide the handle 42 with several gripping surfaces or with no gripping surfaces. During a suturing procedure, the anchor 22 is loaded into the opening 46 of the cannula 40 and the surgeon grips the handle 42 and directs the pointed distal end 44 of the cannula to a targeted tissue landmark. Force delivered to the handle 42 in a distal direction will drive the pointed distal end 44 of the cannula 40 into the tissue, such that a subsequent withdrawal of the introducer 24 in a proximal direction will allow the introducer 24 to exit the tissue. The spine 34 (and the eyelet 36) engages with the tissue, thus leaving the anchor 22 engaged with and deposited in the tissue after the cannula 40 is withdrawn.

In one embodiment, the introducer 24 includes a pair of cannulas, including a second cannula 50 having a pointed distal end 54 and an opening 56 formed in the cannula 50. The second cannula 50 is provided to receive a second, separate anchor. With this in mind, a second anchor 62 is provided having a body 70 having a pointed leading end 72, a spine 74 projecting radially from the body 70, an eyelet 76 attached to a trailing end 77 of the body 70, and a second length of suture 78 attached to the eyelet 76. In this embodiment, the introducer 24 is operable to deliver the first anchor 22 out of the first cannula 40 and to subsequently deliver the second anchor 62 out of the second cannula 50. The gripping surface 48 is configured to allow the translation or rotation of the instrument to selectively move each of the cannulas 40, 50 to a forward facing proximal position.

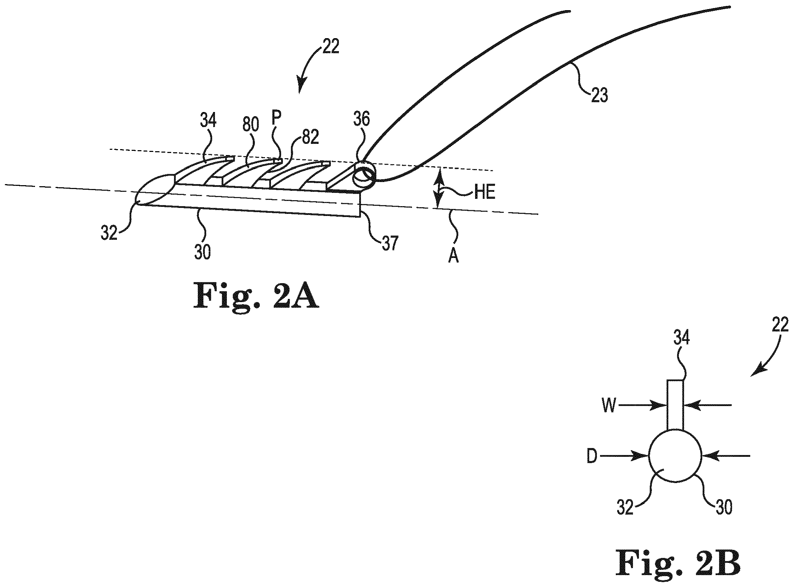

Figure 2A is a perspective view of the anchor 22 and Figure 2B is an end view of the anchor 22. The anchor 22 includes multiple spines 34 extending from the body 30. The spines 34 project radially away from a center longitudinal axis A of the body 30, with each spine 34 shaped as a shark fin having a curved leading edge 80 that meets with a curved trailing edge 82 at a point P. The curved leading edge 80 is oriented to diverge away from the pointed leading end 32 of the body 30 to allow the anchor 22 to glide into tissue and prevent the anchor from pulling out of the tissue. Although three spines 34 and one eyelet 36 are illustrated, the anchor 22 is also suitably provided with more than three spines 34.

The eyelet 36 projects radially away from the center longitudinal axis A of the body 30 and as such is also configured to engage with tissue. For example, the eyelet 36 is provided with a height HE that is substantially equal to the height of the spines 34 (the distance that the point P is away from the center axis A). The eyelet has a width substantially equal to the width W of the spine 34.

The body 30 of the anchor 22 is substantially circular in lateral cross-section (Figure 2B). The anchor 22 is configured to slide in an entry direction through the tissue, and is shaped to prevent withdrawal of the anchor 22 in the direction that is opposite of the entry direction. The curved leading edge 80 of the shark fin shape of the spines 34 facilitate the easy sliding of the anchor 22 through the tissue in the entry direction, and the curved trailing edge 82 of the spines 34 configure the anchor to resist being pulled out of the tissue in the direction that is opposite of the entry direction. In one embodiment, the body 30 of the anchor 22 has a diameter D, and the spine 34 has a width W that is less than about 25% of the diameter D (Figure 2B).

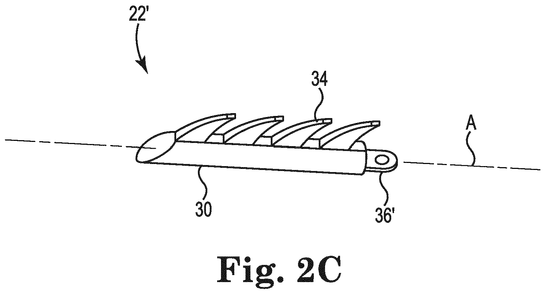

Figure 2C is a perspective view of another example of an anchor 22' not forming part of the invention, provided with an eyelet 36'that is disposed on the center longitudinal axis A of the body 30. The spines 34 of the anchor 22' are provided to engage with tissue, and the eyelet 36' is streamlined to follow the body 30 into the tissue channel that is formed when the anchor 22' is driven into the tissue by the introducer 24 (Figure 1).

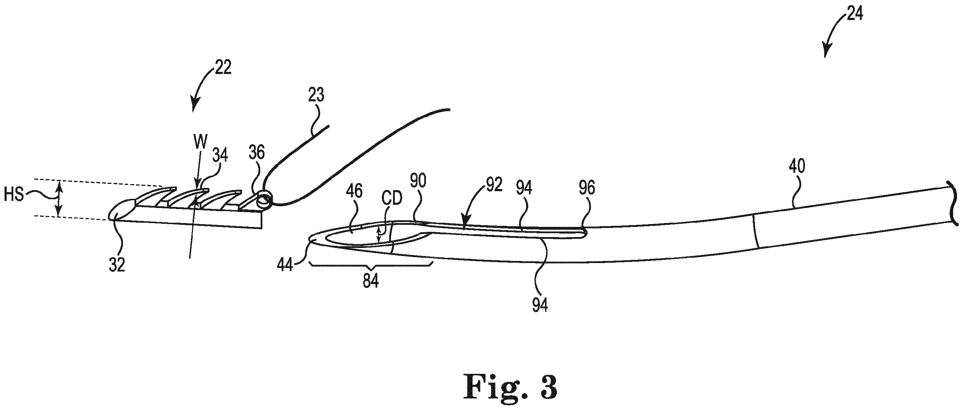

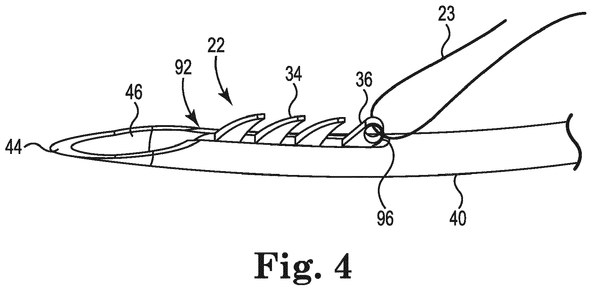

Figure 3 is a perspective view of the anchor 22 positioned for insertion into the cannula 40 of the introducer 24 and Figure 4 is a perspective view of the anchor 22 inserted into the cannula 40. The body 30 of the anchor 22 is sized to slide into the opening 46 (also called a lumen 46) of the cannula 40 with the spine 34 projecting out of the cannula 40. With reference to Figure 3, the inside diameter of the lumen 46 of the cannula 40 provides a cannula diameter CD, and the spine 34 has a height HS that is greater than the cannula diameter CD. The height HS the spine 34 is at least 5% greater than the cannula diameter CD. For example, the height HS of the spine 34 is in the range of 5-100% greater than the cannula diameter CD.

It is acceptable for the height HE (Figure 2A) of the eyelet 36 to be equal to the height HS of the spine 34. It is also acceptable for the height HE (Figure 2A) of the eyelet 36 to be different from and not equal to the height HS of the spine 34.

The cannula 40 includes a tapered distal end portion 84 that tapers to the pointed distal end 44, where the tapered distal end portion 84 provides the cannula 40 with a needle-like point adapted for insertion through tissue. In some applications, the pointed distal end 44 of the cannula 40 is sharp and needle-like and is so configured to enter the periosteum tissue covering a boney surface and glide under the periosteum tissue and over the bone. In this manner, the cannula is configured to deliver the anchor 22 between the periosteum tissue and the bone.

The cannula 40 has a wall 90 that forms or defines the lumen 46 and a slot 92 formed through the wall 90. The slot 92 is proximal of the tapered distal end portion 84 and extends through the wall 90 to communicate with the lumen 46. The slot 92 includes a pair of opposed longitudinal side edges 94 that extend from a proximal lateral edge 96 in a distal direction to the distal end portion 84. The width of the slot between the longitudinal side edges 94 is sized to receive the width W of the spines 34. The cannula diameter CD is sized to receive the diameter D (Figure 2B) of the body 30 of the anchor 22.

With reference to Figure 4, when the anchor 22 is loaded into the cannula 40, the pointed leading end 32 of the body 30 is located proximal of the pointed distal end 44 of the cannula 40, and the spines 34 and the eyelet 36 extend outside of the cannula 40 and are positioned to engage with tissue during implantation of the anchor 22. The proximal lateral edge 96 of the slot 92 is positioned to push against the eyelet 36 and drive the anchor 22 into the tissue. The opposed longitudinal side edges 94 of the slot 92 provide a stanchion that restrains the spines 34 and prevents the anchor 22 from rotating relative to the cannula 40. The spines 34 and the eyelet 36 slide in a longitudinal direction relative to the slot 92 to allow the cannula 40 to be removed from the tissue while leaving the anchor 22 implanted.

Suitable materials for fabricating the anchor 22 include plastics, or metal, or sintered material. One suitable material for fabricating the anchor 22 is polypropylene. Another suitable material for fabricating the anchor 22 is a bioabsorbable polymer that configures the anchor 22 to be absorbed into the body over a period of several weeks.

Suitable materials for fabricating the length of suture 23 include bio-inert components that do not bioabsorb, or bioabsorbable components that are configured to be absorbed or resorbed by the body. One suitable material for fabricating the length of suture 23 is polypropylene. Other suitable materials for fabricating the length of suture 23 include dissolvable sutures available from Ethicon™, a J&J Company located in Somerville, NJ, and include Monocryl™ (polyglycaprone 25) sutures, coated Vicryl™ (polyglactin 910) sutures, Ethicon Plus™ Sutures, or polydioxanone sutures as examples.

Suitable materials for fabricating the cannula 40 and include plastics or metal. One suitable material for fabricating the cannula 40 is stainless steel. Other suitable materials are acceptable.

With reference to Figure 1, the anchor 22 is useful for fixating a support material within a patient's body. The introducer 24 is sized to place the anchors 22 through a single incision and into the periosteum tissue that covers the pubic bone, examples of which are described below.

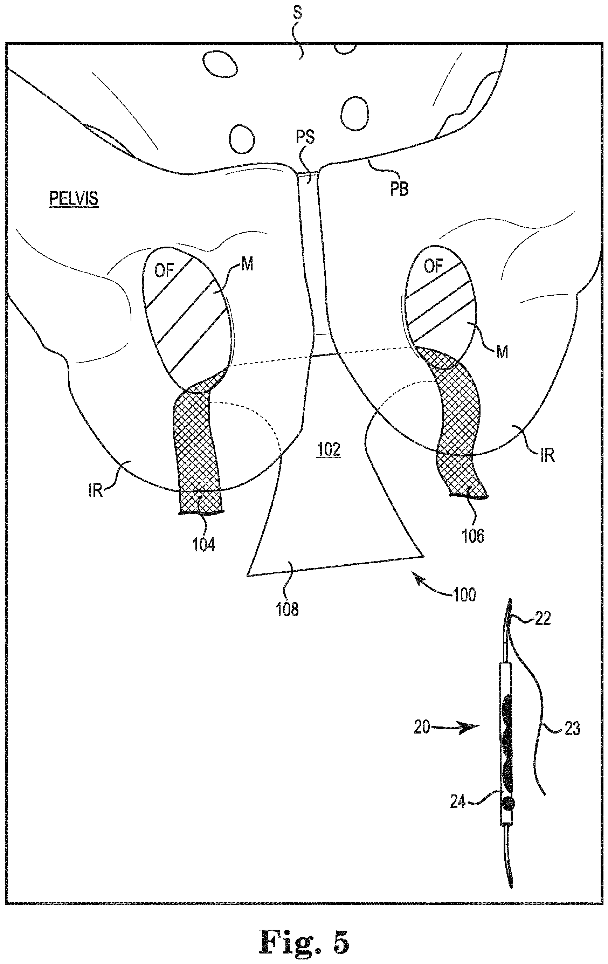

Figure 5 is a schematic view of one embodiment of a support 100 attachable to a pelvis of a patient. Figure 5 provides an anterior view of the pelvis with the sacrum S located in a posterior portion of the view, with the pubic symphysis PS centered relative to the pubic bone PB, and an obturator foramen OF on each bilateral side of the pelvis. Each obturator foramen OF provides an opening or a window that is covered by a membrane M. Nerves and arteries traverse the upper reaches of the obturator foramen OF. The membrane M generally includes several layers of muscle and at least one layer of ligament-like tissue that connects the muscles in the membrane M to the pelvis. The ischial pubic ramus IR is located inferior to the pubic bone PB and the obturator foramen OF.

The support 100 is provided to elevate and compress the male urethra and includes a body 102, a first arm 104 extending from the body 102, a second arm 106 extending from the body 102, and a pre-pubic portion 108 that is oriented in a generally orthogonal position relative to the arms 104, 106. The illustrated embodiment is a two-arm device.

Suitable materials for fabricating the support 100 include porous materials that allow tissue ingrowth throughout the support structure to anchor the support 100 in the body after implantation and healing. Suitable such porous materials include autograft material (the patient's own tissue), allograft material (tissue from a cadaver), xenograft material (tissue from another species), or synthetic materials such as woven fabrics, meshes, nonwoven fabrics, meshes, fibrillated fibers, or spun and fibrillated fibers that are provided with voids (pores) configured to allow tissue ingrowth into the support 100. The pores are generally larger, on average, than 75 µm.

The support 100 is attached to the pelvis with each arm 104, 106 inserted into one of the respective obturator foramen OF, and with the pre-pubic portion 108 attached to the periosteum tissue that lines the exterior of the pubic bone PB. The following surgical procedure is one example of the suitable implantation of the support 100 into a male patient.

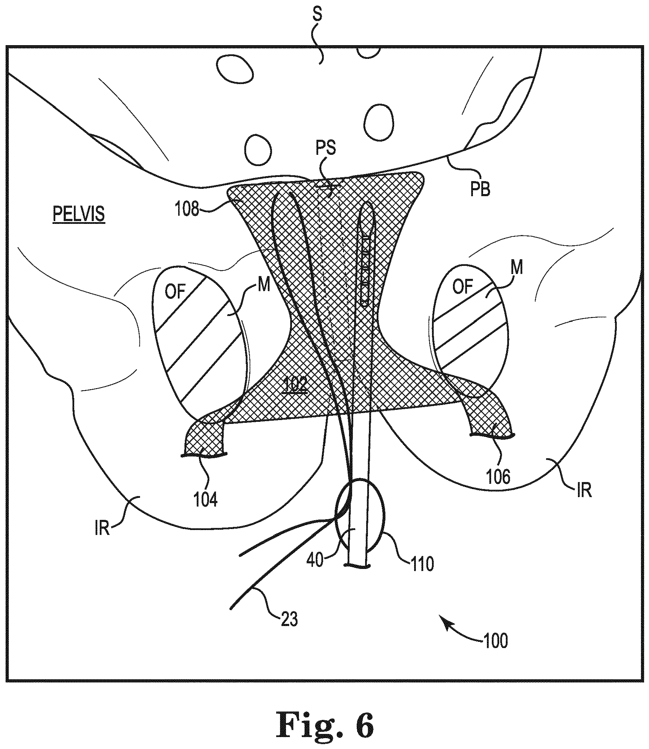

The patient is positioned on a surgical operating table in a lithotomy, or modified lithotomy position, and is anesthetized. A vertical midline perineal incision 110 (see Figure 6) is formed between the scrotum and the anus. Tissue is dissected to expose the bulbous muscle around the urethra. A suitable tool is used to direct the arm 104 into and through the first obturator foramen OF, and this procedure is repeated on the contralateral side to place the arm 106 into and through the second obturator foramen OF.

One suitable approach of placing the arms 104, 106 through the obturator foramen OF is described as an "outside-in" approach. The outside-in approach includes directing a needle or other device through the skin of the groin area of the patient external of the obturator foramen OF along a curved path through the membrane M and around the ischial pubic ramus R such that the tool exits the midline perineal incision 110. One of the arms 104, 106 is attached to the tool, and the tool is withdrawn along its curved pathway back around the ischial pubic ramus IR, through the membrane M, out of the obturator foramen OF, and out of the skin at the groin area. In this manner, each arm 104, 106 is directed through and placed in one of the obturator foramen OF. The arms 104, 106 are trimmed to a subcutaneous level. A holding stitch is placed to hold the arm 104, 106 relative to the groin tissue, as determined by the surgeon.

A different approach is the "inside-out" approach in which the needle or tool is coupled to the support and directed from the perineal incision (inside) outward to the skin at the groin area (outside). Placement of the arms 104, 106 with the inside-out approach is also acceptable.

One acceptable single incision approach includes the formation of a single (exactly one) incision in the urogenital triangle. Tissue is dissected distal the incision to access the urethra and the pelvis. The arms 104, 106 of the support 100 are directed into the single incision and anchored to the membrane M of the obturator foramen OF, for example with the anchor 22 (Figure 1). The pre-pubic portion 108 is inserted into the single incision and fixed to the periosteum tissue over the pubic bone PB by the anchor 22 as delivered by the introducer 24. In this manner, a treatment for urinary incontinence is provided to the patient by forming exactly and only one incision and implanting the support 100 through that single incision.

Figure 6 is a schematic view of the surgical system 20 employed to fixate the pre-pubic portion 108 of the support 100 to the periosteum tissue of the pubic bone PB. The cannula 40 of the introducer 24 is inserted into the perineal incision 110 and directed to the pubic bone PB anterior to the pelvis.

In one suitable approach, the anchor 22 is driven through the material of the support 100 and into the periosteum tissue that covers the pubic bone PB. The cannula 40 pierces the periosteum tissue and slides along the bone of the pelvis without entering or penetrating the bone. The anchor 22 is engaged under the periosteum tissue and the suture 23 extends through the support 100 out through the perineal incision 110. The surgeon, depending upon surgeon preference, will place at least one anchor 22 through the pre-pubic portion 108 an each side of the pubic symphysis PS. The suture 23 extends from each anchor out through the perineal incision 110 and is available for subsequent tying or other termination.

In a different suitable approach, the anchor 22 is loaded into the introducer 24 and the cannula 40 is introduced in the perineal incision 110 up to the pubic bone PB anterior to the pelvis. The introducer 24 is employed to drive the anchor 22 under the periosteum tissue of the pubic bone PB and the cannula 40 is withdrawn through the perineal incision 110. The suture 23 trails behind the anchor 22 and exits the body at the incision 110. An end of the suture 23 is inserted through the pre-pubic portion 108 of the support 100, and the pre-pubic portion 108 is guided along the suture 23, through the incision 110, and up to the pubic bone PB. Thereafter, the suture 23 is tied or terminated to hold the pre-pubic portion 108 against the pubic bone.

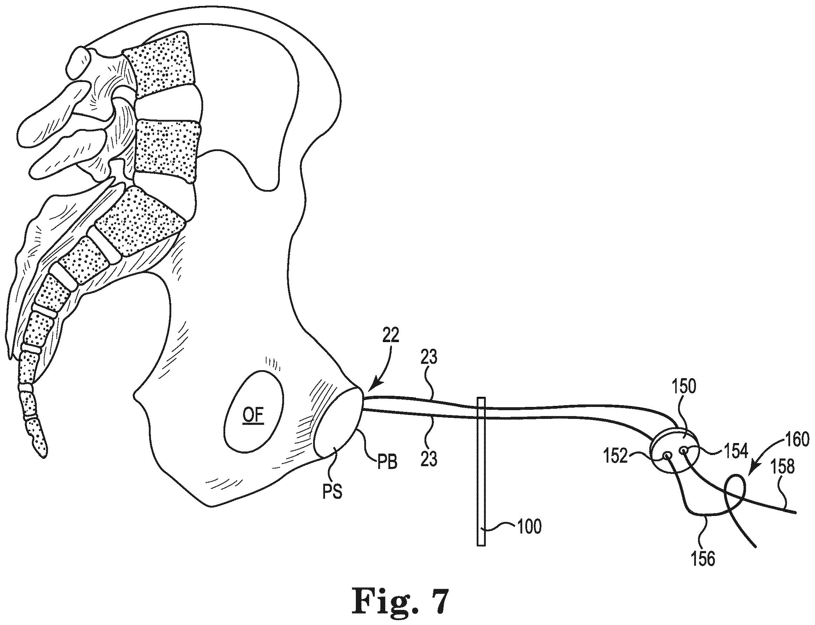

Figure 7 is a schematic view of the anchor 22 secured to the periosteum tissue and the support 100 secured to the suture 23. In one embodiment, the system 20 described above includes a stopper 150 that is attached to the suture 23, where the stopper 150 is configured to slide along the suture 23 and direct the support 100 into the patient's body and against the tissue. In one embodiment, the stopper 150 has a first orifice 152 and a second orifice 154. One or more of the anchors 22 is engaged with the periosteum tissue of the pubic bone PB, and a first end 156 of the suture 23 extends from the anchor 22 through the first orifice 152, and a second end 158 of the suture 23 extends to the second orifice 154. The stopper 150 slides along the suture 23 and is operable to push or otherwise deliver the support 100 against the pubic bone PB. In one embodiment, a slip knot 160 or other termination device is provided to tie the suture 23 against the stopper 150 after the stopper 150 and the support 100 has been delivered to the pubic bone PB. The stopper 150 is located between the anchor 22 and the slip knot 160.

Suitable materials for fabricating the stopper 150 include plastics or metal. One suitable material for fabricating the stopper 150 includes polypropylene. Another suitable material for fabricating the stopper 150 includes stainless steel. In one embodiment, the stopper 150 is fabricated to be bioabsorbable.

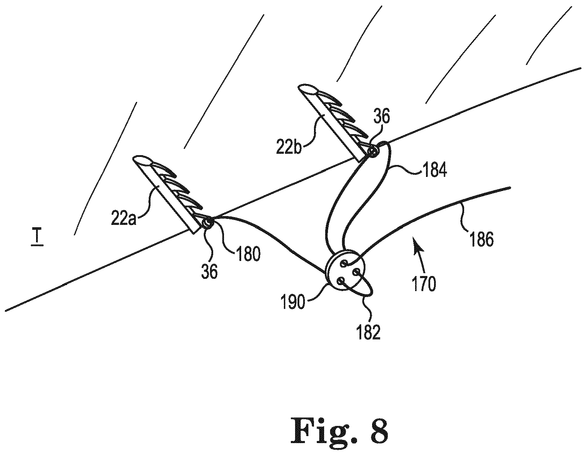

Figure 8 is a schematic view of two anchors 22 secured to tissue T and coupled with a suture 170. The anchors include a first anchor 22a and a second anchor 22b. The anchors 22 are engaged with the tissue T, for example through the use of the introducer 24 (Figure 1). A suture 170 is provided having a first end 180 terminated to the eyelet 36 of the anchor 22a, a mid-portion 182 of the suture located between the first anchor 22a and the second anchor 22b, and a portion 184 of the suture in sliding engagement with the eyelet 36 of the second anchor 22b. A free end 186 of the suture 170 is provided, and pulling on the free end 186 of the suture 170 cinches the mid-portion 182 of the suture between the first anchor 22a and the second anchor 22b. In one embodiment, a slide knot 190 or sliding engagement feature 190 is coupled to the suture 170 and is so configured to secure or lock the mid-portion 182 of the suture in a desired position relative to the anchors 22. The slide knot 190 operates to cinch the suture 170 tightly against the support 100 (Figure 6) against the tissue T.

Some male incontinence treatment devices have several arms, including some form of arms that traverse the obturator foramen and other arms that are implanted anterior to the pubic bone (called pre-pubic arms). The pre-pubic arms are tunneled anterior to the pelvis and exit the skin of the abdomen.

In contrast, embodiments of the system described above provide a support with two arms that are A) secured to the periosteum alongside the obturator foramen or B) secured to the membrane M covering the obturator foramen or C) secured through the obturator foramen and a system 20 to attach a portion of the support directly and efficiently to the periosteum tissue over the pubic bone. The system obviates the use of additional pre-pubic arms that are tunneled under and affixed to the skin. The system is easier to implant and reduces the amount of time that the patient is in the operating room.

Although specific embodiments have been illustrated and described herein, it will be appreciated by those of ordinary skill in the art that a variety of alternate and/or equivalent implementations may be substituted for the specific embodiments shown and described without departing from the scope of the present invention. This application is intended to cover any adaptations or variations of medical devices as discussed herein. Therefore, it is intended that this invention is limited only by its claims.

## Claims

1. A surgical system (20) comprising:
an anchor (22); and
an introducer (24) provided to deliver the anchor (22) into tissue;
wherein the anchor (22) includes a body (30) having a pointed leading end (32) that is configured to pierce the tissue, a plurality of spines (34) projecting radially away from the body (30) having a width and a height configured to allow the spines (34) to engage with the tissue, an eyelet (36) attached to a trailing end (37) of the body (30), and a length of suture (23) attached to the eyelet (36);
wherein the introducer (24) includes a cannula (40), the cannula having a pointed distal end (44), an opening (46) formed in the cannula (40) that is sized to receive the body (30) of the anchor (22), and a slot (92) formed through a wall (90) of the cannula (40) at a distal end portion (84) of the cannula (40) and sized to receive the width of the spines (34);
wherein when readied for use, the anchor (22) is secured in the introducer (24) with the body (30) of the anchor (22) inserted into the opening (46) of the cannula (40) with the pointed leading end (32) of the body (30) located proximal of the pointed distal end (44) of the cannula (40), and with the spines (34) of the anchor (22) inserted into the slot (92) of the cannula (40) such that the anchor (22) does not rotate relative to the cannula (40), wherein each of the plurality of spines (34) is shaped as a shark fin with a curved leading edge (80) meeting with a curved trailing edge (82) at a point, with the curved leading edge (80) diverging away from the pointed leading end (32) of the body (30), and wherein the eyelet (36) projects radially away from a center longitudinal axis of the body (30) to extend outside of the cannula (40) through the slot (92) and as such is also configured to engage with the tissue during implantation of the anchor and has a width substantially equal to the width of the spines (34).

2. The surgical system of claim 1, wherein the body (30) of the anchor (22) is substantially circular in lateral cross-section.

3. The surgical system of claim 2, wherein the body (30) of the anchor (22) has a diameter and the width of the spine (34) is less than 25% of the diameter.

4. The surgical system of claim 1, wherein the cannula (40) has a cannula diameter and a height of the spine (34) is at least 50% greater than the cannula diameter.

5. The surgical system of claim 1, wherein the introducer (24) has a handle (42) having a first end opposite a second end and the cannula (40) includes a first cannula extending from the first end of the handle (42) and a second cannula extending from the second end of the handle (42).

6. The surgical system of claim 1, further comprising:
a stopper (150) attached to the length of suture (23);
wherein the length of suture (23) includes first strand (156) inserted into a first orifice (152) of the stopper (150) and a second strand (158) inserted into a second orifice (154) of the stopper (150).

7. The surgical system of claim 6, further comprising:
a slip knot (160) tied in the first and second strands (156, 158) of the length of suture (23), with the stopper (150) located between the anchor (22) and the slip knot (160).

8. The surgical system of claim 7, further comprising:
a porous support material (100), the anchor (22) insertable through the porous support material (100) to locate the anchor (22) on a first side of the porous support material (100) and to locate the stopper (160) on a second opposite side of the first side of the porous support material (100).

9. The surgical system of claim 1, comprising a first anchor (22a) having a first eyelet (36) and a second anchor (22b) having a second eyelet (36), with a first end (180) of the length of suture (170) fixed to the first eyelet (36) of the first anchor (22a), a mid-portion (182) of the length of suture located between the first (22a) and second (22b) anchors, and the length of suture (170) movable in sliding engagement with the second eyelet (36) of the second anchor (22b).

10. The surgical system of claim 9, further comprising:
a slide knot (190) formed from tying a first end portion (180) of the length of suture (170) to the mid-portion (182) of the length of suture;
wherein the slide knot (190) operates to cinch the length of suture (170) tight against a support that is secured to the tissue by the first and second anchors (22a, 22b).

11. The surgical system of claim 1, wherein the eyelet (36) is provided with a height that is substantially equal to the height of the spines (34).

12. The surgical system of claim 1, wherein the anchor (22) is made from a bioabsorbable polymer that configures the anchor (22) to be absorbed into the body over a period of several weeks.

13. The surgical system of claim 5, wherein the handle (42) comprises a gripping surface (48) formed on at least one side of the handle (42).

14. The surgical system of claim 13, wherein the second cannula (50) comprises a pointed distal end (54) and an opening (56) formed in the cannula (50).

15. The surgical system of claim 14, wherein the gripping surface (48) is configured to allow the translation or rotation of the instrument to selectively move each of the cannulas (40, 50) to a forward facing proximal position.

## Patentansprüche

1. Chirurgisches System (20), umfassend:
einen Anker (22); und
eine Einführvorrichtung (24), die zur Abgabe des Ankers (22) in Gewebe vorgesehen ist;
wobei der Anker (22) einen Körper (30) mit einem spitz zulaufenden Vorderende (32), das zum Einstechen in das Gewebe ausgelegt ist, eine Vielzahl von Dornen (34), die vom Körper (30) radial weg ragen, mit einer Breite und einer Höhe, die dazu ausgelegt sind, einen Eingriff der Dorne (34) mit dem Gewebe zu gestatten, eine Öse (36), die an einem Hinterende (37) des Körpers (30) befestigt ist, und eine Länge eines Nahtmaterials (23), das an der Öse (36) befestigt ist, aufweist;
wobei die Einführvorrichtung (24) eine Kanüle (40) aufweist, wobei die Kanüle ein spitz zulaufendes distales Ende (44), eine in der Kanüle (40) ausgebildete Öffnung (46) mit einer Größe zur Aufnahme des Körpers (30) des Ankers (22) und einen durch eine Wand (90) der Kanüle (40) an einem distalen Endabschnitt (84) der Kanüle (40) ausgebildeten Schlitz (92) mit einer Größe zur Aufnahme der Breite der Dorne (34) aufweist;
wobei der Anker (22), wenn er zur Benutzung bereit gemacht wird, in der Einführvorrichtung (24) befestigt wird, wobei der Körper (30) des Ankers (22) in die Öffnung (46) der Kanüle (40) eingeführt wird, wobei das spitz zulaufende Vorderende (32) des Körpers (30) proximal vom spitz zulaufenden distalen Ende (44) der Kanüle (40) angeordnet ist und wobei die Dorne (34) des Ankers (22) derart in den Schlitz (92) der Kanüle (40) eingeführt werden, dass sich der Anker (22) bezüglich der Kanüle (40) nicht dreht,
wobei jeder der Vielzahl von Dornen (34) als Haifischflosse geformt ist, wobei eine gebogene Vorderkante (80) an einem Punkt auf eine gebogene Hinterkante (82) trifft, wobei die gebogene Vorderkante (80) von dem spitz zulaufenden Vorderende (32) des Körpers (30) weg divergiert und wobei die Öse (36) radial von einer Mittellängsachse des Körpers (30) weg ragt, um sich außerhalb der Kanüle (40) durch den Schlitz (92) zu erstrecken und somit auch für einen Eingriff mit dem Gewebe während der Implantation des Ankers ausgelegt ist und eine Breite aufweist, die der Breite der Dorne (34) im Wesentlichen gleicht.

2. Chirurgisches System nach Anspruch 1, wobei der Körper (30) des Ankers (22) einen im Wesentlichen kreisförmigen seitlichen Querschnitt aufweist.

3. Chirurgisches System nach Anspruch 2, wobei der Körper (30) des Ankers (22) einen Durchmesser aufweist und die Breite des Dorns (34) weniger als 25% des Durchmessers beträgt.

4. Chirurgisches System nach Anspruch 1, wobei die Kanüle (40) einen Kanülendurchmesser aufweist und eine Höhe des Dorns (34) mindestens 50% größer als der Kanülendurchmesser ist.

5. Chirurgisches System nach Anspruch 1, wobei die Einführvorrichtung (24) einen Griff (42) mit einem ersten Ende gegenüber einem zweiten Ende aufweist und die Kanüle (40) eine erste Kanüle, die sich vom ersten Ende des Griffs (42) erstreckt, und eine zweite Kanüle, die sich vom zweiten Ende des Griffs (42) erstreckt, aufweist.

6. Chirurgisches System nach Anspruch 1, ferner umfassend:
einen an der Nahtmateriallänge (23) befestigten Stopper (150);
wobei die Nahtmateriallänge (23) einen ersten Strang (156), der in eine erste Öffnung (152) des Stoppers (150) eingeführt ist, und einen zweiten Strang (158), der in eine zweite Öffnung (154) des Stoppers (150) eingeführt ist, aufweist.

7. Chirurgisches System nach Anspruch 6, ferner umfassend:
einen Zugknoten (160), der in den ersten und zweiten Strängen (156, 158) der Nahtmateriallänge (23) geknotet ist, wobei der Stopper (150) zwischen dem Anker (22) und dem Zugknoten (160) angeordnet ist.

8. Chirurgisches System nach Anspruch 7, ferner umfassend:
ein poröses Stützmaterial (100), wobei der Anker (22) durch das poröse Stützmaterial (100) einführbar ist, um den Anker (22) auf einer ersten Seite des porösen Stützmaterials (100) anzuordnen und den Stopper (160) auf einer zweiten, gegenüberliegenden Seite der ersten Seite des porösen Stützmaterials (100) anzuordnen.

9. Chirurgisches System nach Anspruch 1, umfassend einen ersten Anker (22a) mit einer ersten Öse (36) und einen zweiten Anker (22b) mit einer zweiten Öse (36), wobei ein erstes Ende (180) der Nahtmateriallänge (170) an der ersten Öse (36) des ersten Ankers (22a) befestigt ist, ein mittlerer Abschnitt (182) der Nahtmateriallänge zwischen dem ersten (22a) und dem zweiten (22b) Anker angeordnet ist und die Nahtmateriallänge (170) in Gleiteingriff mit der zweiten Öse (36) des zweiten Ankers (22b) bewegbar ist.

10. Chirurgisches System nach Anspruch 9, ferner umfassend:
einen Zugknoten (190), der durch Verknoten eines ersten Endabschnitts (180) der Nahtmateriallänge (170) am mittleren Abschnitt (182) der Nahtmateriallänge gebildet wird;
wobei der Zugknoten (190) dazu dient, die Nahtmateriallänge (170) fest gegen eine Stütze zusammenzuziehen, die von dem ersten und dem zweiten Anker (22a, 22b) am Gewebe befestigt wird.

11. Chirurgisches System nach Anspruch 1, wobei die Öse (36) mit einer Höhe ausgestattet ist, die im Wesentlichen der Höhe der Dorne (34) gleicht.

12. Chirurgisches System nach Anspruch 1, wobei der Anker (22) aus einem bioresorbierbaren Polymer gefertigt ist, das den Anker (22) so konfiguriert, dass er über eine Zeitspanne von mehreren Wochen in den Körper resorbiert wird.

13. Chirurgisches System nach Anspruch 5, wobei der Griff (42) eine Greiffläche (48) umfasst, die auf mindestens einer Seite des Griffs (42) ausgebildet ist.

14. Chirurgisches System nach Anspruch 13, wobei die zweite Kanüle (50) ein spitz zulaufendes distales Ende (54) und eine in der Kanüle (50) ausgebildete Öffnung (56) umfasst.

15. Chirurgisches System nach Anspruch 14, wobei die Greiffläche (48) dazu ausgelegt ist, die Verschiebung oder Drehung des Instruments zu gestatten, um jede der Kanülen (40, 50) selektiv in eine nach vorne weisende proximale Position zu bewegen.

## Revendications

1. Système chirurgical (20) comprenant :
un élément d'ancrage (22) ; et
un dispositif d'introduction (24) conçu pour amener l'élément d'ancrage (22) dans un tissu ;
l'élément d'ancrage (22) comprenant un corps (30) ayant une extrémité avant pointue (32) conçu pour percer le tissu, une pluralité d'arêtes (34) se projetant radialement à l'opposé du corps (30) et ayant une largeur et une hauteur conçues pour permettre aux arêtes (34) de prendre appui sur le tissu, un oeillet (36) fixé à une extrémité arrière (37) du corps (30), et une longueur de suture (23) fixée à l'oeillet (36) ; le dispositif d'introduction (24) comprenant une canule (40), la canule ayant une extrémité distale pointue (44), une ouverture (46) formée dans la canule (40) et dimensionnée pour recevoir le corps (30) de l'élément d'ancrage (22), et une rainure (92) formée dans une paroi (90) de la canule (40) au niveau d'une partie extrême distale (84) de la canule (40) et dimensionnée pour recevoir la largeur des arêtes (34) ;
l'élément d'ancrage (22), lorsqu'il est prêt à être utilisé, étant fixé dans le dispositif d'introduction (24) avec le corps (30) de l'élément d'ancrage (22) inséré dans l'ouverture (46) de la canule (40) avec l'extrémité avant pointue (32) du corps (30) située à proximité de l'extrémité distale pointue (44) de la canule (40), et avec les arêtes (34) de l'élément d'ancrage (22) insérées dans la rainure (92) de la canule (40) de sorte que l'élément d'ancrage (22) ne pivote pas par rapport à la canule (40) ;
chaque arête de la pluralité d'arêtes (34) présentant la forme d'un aileron de requin avec un bord avant courbé (80) rencontrant un bord arrière courbé (82) au niveau d'un point, le bord avant courbé (80) s'écartant de l'extrémité avant pointue (32) du corps (30), et l'oeillet (36) se projetant radialement à l'opposé d'un axe longitudinal central du corps (30) pour s'étendre hors de la canule (40) par la rainure (92) et conçu comme tel pour prendre appui sur le tissu pendant l'implantation de l'élément d'ancrage, et ayant une largeur sensiblement égale à la largeur des arêtes (34).

2. Système chirurgical selon la revendication 1, dans lequel le corps (30) de l'élément d'ancrage (22) est sensiblement circulaire en coupe latérale.

3. Système chirurgical selon la revendication 2, dans lequel le corps (30) de l'élément d'ancrage (22) a un certain diamètre, et la largeur de l'arête (34) est inférieure à 25 % du diamètre.

4. Système chirurgical selon la revendication 1, dans lequel la canule (40) a un certain diamètre de canule, et une hauteur de l'arête (34) est supérieure d'au moins 50 % au diamètre de canule.

5. Système chirurgical selon la revendication 1, dans lequel le dispositif d'introduction (24) comporte une poignée (42) ayant une première extrémité opposée à une seconde extrémité, et la canule (40) comprend une première canule s'étendant à partir de la première extrémité de la poignée (42) et une seconde canule s'étendant à partir de la seconde extrémité de la poignée (42).

6. Système chirurgical selon la revendication 1, comprenant en outre :
un élément d'arrêt (150) fixé à la longueur de suture (23) ;
la longueur de suture (23) comprenant un premier brin (156) inséré dans un premier orifice (152) de l'élément d'arrêt (150) et un second brin (158) inséré dans un second orifice (154) de l'élément d'arrêt (150).

7. Système chirurgical selon la revendication 6, comprenant en outre :
un noeud coulant (160) lié sur les premier et second brins (156, 158) de la longueur de suture (23), avec l'élément d'arrêt (150) situé entre l'élément d'ancrage (22) et le noeud coulant (160).

8. Système chirurgical selon la revendication 7, comprenant en outre :
un matériau de support poreux (100), l'élément d'ancrage (22) pouvant être inséré à travers le matériau de support poreux (100) pour situer l'élément d'ancrage (22) sur un premier côté du matériau de support poreux (100) et pour situer l'élément d'arrêt (160) sur un second côté opposé au premier côté du matériau de support poreux (100).

9. Système chirurgical selon la revendication 1, comprenant un premier élément d'ancrage (22a) ayant un premier oeillet (36) et un second élément d'ancrage (22b) ayant un second oeillet (36), avec une première extrémité (180) de la longueur de suture (170) fixée au premier oeillet (36) du premier élément d'ancrage (22a), une partie médiane (182) de la longueur de suture située entre les premier (22a) et second (22b) éléments d'ancrage, et la longueur de suture (170) mobile en contact coulissant avec le second oeillet (36) du second élément d'ancrage (22b).

10. Système chirurgical selon la revendication 9, comprenant en outre :
un noeud coulissant (190) formé en liant une première partie extrême (180) de la longueur de suture (170) à la partie médiane (182) de la longueur de suture ;
le noeud coulissant (190) servant à serrer la longueur de suture (170) contre un support qui est fixé au tissu par les premier et second éléments d'ancrage (22a, 22b).

11. Système chirurgical selon la revendication 1, dans lequel l'oeillet (36) a une hauteur qui est sensiblement égale à la hauteur des arêtes (34).

12. Système chirurgical selon la revendication 1, dans lequel l'élément d'ancrage (22) est constitué d'un polymère bioabsorbable qui permet à l'élément d'ancrage (22) d'être absorbé dans le corps sur une période de plusieurs semaines.

13. Système chirurgical selon la revendication 5, dans lequel la poignée (42) comprend une surface de saisie (48) formée sur au moins un côté de la poignée (42).

14. Système chirurgical selon la revendication 13, dans lequel la seconde canule (50) comprend une extrémité distale pointue (54) et une ouverture (56) formée dans la canule (50).

15. Système chirurgical selon la revendication 14, dans lequel la surface de saisie (48) est conçue pour permettre la translation ou la rotation de l'instrument pour déplacer de manière sélective chacune des canules (40, 50) dans une position proximale orientée vers l'avant.
